# EUROPEAN PATENT APPLICATION

(11) **EP 4 650 048 A1**
(43) Date of publication of application: **19.11.2025**
(21) Application number: 24844374.9
(22) Date of filing: 27.05.2024
(51) Int. Cl.: B01J 31/34, C07D 307/48

(54) **DOUBLE-ACID CATALYST AND METHOD FOR PREPARING FURFURAL BY CATALYZING HEMICELLULOSE OR XYLOSE**

(30) Priority: 23.07.2023 CN 202310907836
(71) Applicant: Zhejiang Huakang Pharmaceutical Co., Ltd., Quzhou, Zhejiang 324302 (CN); Nanjing University, Nanjing, Jiangsu 210023 (CN)
(72) Inventor: WU, Youting, Nanjing, Jiangsu 210023 (CN); LI, Mian, Santa Clara (US); XU, Guangzhi, Nanjing, Jiangsu 210023 (CN); LIAO, Chengjun, Quzhou, Zhejiang 324302 (CN); HU, Changhui, Quzhou, Zhejiang 324302 (CN); HU, Xingbang, Nanjing, Jiangsu 210023 (CN); CHENG, Xinping, Quzhou, Zhejiang 324302 (CN); TU, Zhuoheng, Nanjing, Jiangsu 210023 (CN); CHEN, Deshui, Quzhou, Zhejiang 324302 (CN)
(74) Representative: Dai, Simin
(86) International application number: PCT/CN2024/095618
(87) International publication number: WO 2025/020679

(57) **Abstract**

A bifunctional acidic catalyst and a preparation method of furfural via catalyzing hemicellulose or xylose are provided. The bifunctional acidic catalyst includes choline chloride, Brønsted acid, and Lewis acid in a molar ratio of 1:1:(1-4), and the bifunctional acidic catalyst is in a deep eutectic state. The method includes adding hemicellulose or xylose and the bifunctional acidic catalyst as an aqueous phase into a reactor, followed by adding an oil phase extractant, generating furfural in the reactor through catalytic dehydration and hydrolysis reaction, and continuously extracting the furfural from the aqueous phase by the oil phase extractant during the reaction process until the reaction is ended. The choline chloride, Brønsted acid, and Lewis acid are combined to form a deep eutectic solvent as the bifunctional acidic catalyst, which is used in the process of preparing furfural via catalyzing hemicellulose or xylose, reducing the activation energy required for the reaction and increasing the yield of furfural. In addition, both the aqueous phase and the catalyst in the preparation process may be recycled, which can reduce energy consumption and production cost.

## Description

### CROSS-REFERENCE TO RELATED APPLICATIONS

This application claims priority to Chinese Patent Application No. 202310907836.9, filed on July 23, 2023, the entire contents of which are hereby incorporated by reference.

### TECHNICAL FIELD

The present disclosure relates to the technical field of furfural preparation, and in particular, to bifunctional acidic catalysts and preparation methods of furfural via catalyzing hemicellulose or xylose.

### BACKGROUND

Lignocellulosic biomass is a renewable carbon resource abundant in agricultural wastes, municipal solid wastes, and algae. Lignocellulosic biomass is mainly composed of cellulose, hemicellulose, and lignin. Hemicellulose is primarily obtained by highly branching and polymerization of pentose sugars (e.g., xylans and arabinoxylans). In the presence of an acidic catalyst, hemicellulose is first depolymerized to monosaccharides (e.g., xylose and arabinose) and then further dehydrated to generate furfural.

Furfural is an important renewable platform chemical widely used in the production of various furan derivatives. Furfural can also undergo condensation reaction with aldehydes and ketones to form oxygenated intermediates, which are subsequently subjected to hydrodeoxygenation to produce liquid alkanes used directly as liquid fuels. While furfural has significant potential in the chemical industry, current production processes face challenges such as a low yield and environmental pollution. To further reduce the cost of furfural production, it is essential to develop a sustainable production process.

Currently, the catalysts used for furfural preparation by dehydration of hemicellulose or xylose include zeolites, solid acid catalysts, and ionic liquid catalysts. On the one hand, zeolite and solid acid catalysts involve a complicated preparation process with a high activation energy and corrosion of chemical equipment, which is not conducive to long term production. On the other hand, the ionic liquid catalysts operate in the homogeneous reaction system during the catalytic process, making it difficult to separate the catalyst from the reaction system after ending the reaction, resulting in resource waste.

In addition, the existing process for preparing furfural from biomass raw material uses a deep eutectic solvent composed of choline chloride and Lewis acid as a catalyst. Although the process achieves relatively a high yield of furfural, the reaction temperature is very high, basically exceeding 180°C. Other process uses a deep eutectic solvent composed of choline chloride and Brønsted acid as a catalyst, the reaction temperature is low, generally not exceeding 150°C, but the yield of furfural is also low. Another process uses a mixture of Lewis acid and Brønsted acid as a catalyst, the process achieves a high yield of furfural, and the reaction temperature is moderate. However, the catalyst is difficult to separate, resulting in resource waste.

Therefore, there is a need to develop a low-cost, highly catalytically active, and sustainable catalyst for the preparation of furfural by the dehydration of hemicellulose or xylose.

### SUMMARY

The technical problem to be solved by the present disclosure aims to provide a bifunctional acidic catalyst and a preparation method of furfural via catalyzing hemicellulose or xylose. The present disclosure employs a choline chloride combined with Lewis acid and Brønsted acid to form a deep eutectic solvent as a bifunctional acidic catalyst, which is used in the process of preparing furfural via catalyzing hemicellulose or xylose, reducing the activation energy required for the reaction and increasing the yield of furfural. Additionally, both the aqueous phase and the catalyst in the preparation process may be recycled, which reduces energy consumption and production cost.

One or more embodiments of the present disclosure provide a bifunctional acidic catalyst including choline chloride, Brønsted acid, and Lewis acid in a molar ratio of 1:1:(1-4), the bifunctional acidic catalyst being in a deep eutectic state.

Further, the Brønsted acid may be any one of oxalic acid, malonic acid, succinic acid, and citric acid.

Further, the Lewis acid may be any one of CrCl₃·6H₂O, AlCl₃·6H₂O, SnCl₄·5H₂O, and FeCl₃·6H₂O.

Further, the bifunctional acidic catalyst may be a deep eutectic solvent in the eutectic state and the deep eutectic solvent is prepared by heating a mixture of 1 mol choline chloride, 1 mol oxalic acid, and 1 mol CrCl₃·6H₂O at 70°C for 6 h.

One or more embodiments of the present disclosure provide a preparation method of furfural via catalyzing hemicellulose or xylose. The preparation method may include adding hemicellulose or xylose and the bifunctional acidic catalyst as an aqueous phase to a reactor, followed by adding an oil phase extractant capable of extracting furfural, generating furfural in the reactor through catalytic dehydration and hydrolysis reaction, and continuously extracting the furfural from the aqueous phase during the reaction process by the oil phase extractant until the reaction is ended.

Further, the oil phase extractant may include any one of methyl isobutyl ketone (MIBK), toluene, tetrahydrofuran (THF), valerolactone, n-butanol, acetophenone, ethyl acetate, and tetrahydronaphthalene.

Further, a mass concentration of the hemicellulose or xylose in the aqueous phase may be within a range of 0.05 g/mL-0.25 g/mL.

Further, a mass concentration of the bifunctional acidic catalyst in the aqueous phase may be within a range of 0.05 g/mL-0.25 g/mL.

Further, an amount of the aqueous phase may be 1/2 to 1/10 of an amount of the oil phase extractant.

Further, a reaction temperature of the catalytic dehydration and the hydrolysis reaction may be within a range of 140°C-160°C, and a reaction time of the catalytic dehydration and the hydrolysis reaction may be within a range of 30 min-150 min.

Compared with prior art, for the bifunctional acidic catalyst and the preparation method of furfural via catalyzing hemicellulose or xylose in the present disclosure, the bifunctional acidic catalyst includes choline chloride, Brønsted acid, and Lewis acid in a molar ratio within a range of 1:1:(1-4) in a deep eutectic state. The preparation method of furfural via catalyzing hemicellulose or xylose includes adding hemicellulose or xylose and the bifunctional acidic catalyst as an aqueous phase to a reactor, followed by adding an oil phase extractant, generating furfural in the reactor through catalytic dehydration and hydrolysis reaction, and continuously extracting the furfural from the aqueous phase by the oil phase extractant during the reaction process until the reaction is ended. The present disclosure employs choline chloride combined with Lewis acid and Brønsted acid to form a deep eutectic solvent as the bifunctional acidic catalyst, which is used in the process of preparing furfural via catalyzing hemicellulose or xylose, reducing the activation energy required for the reaction, increasing the yield of furfural, and having a mild reaction condition. Additionally, the aqueous phase and the catalyst in the preparation process may be recycled, which reduces energy consumption and production cost.

### BRIEF DESCRIPTION OF THE DRAWINGS

FIG. 1 is a chart illustrating effects of a reaction temperature and a reaction time on a yield of furfural according to example 3 and example 4 of the present disclosure;
FIG. 2 is a chart illustrating effects of a mass concentration of a catalyst B on a yield of furfural according to example 5 of the present disclosure;
FIG. 3 is a chart illustrating effects of a mass concentration of xylose on a yield of furfural according to example 6 of the present disclosure;
FIG. 4 is a chart illustrating effects of a reaction temperature and a reaction time on a yield of furfural according to example 9 and example 10 of the present disclosure; and
FIG. 5 is a chart illustrating effects of a mass concentration of a catalyst B on a yield of furfural according to example 12 in the present disclosure.

### DETAILED DESCRIPTION

In order to make the technical problems to be solved, technical solutions, and beneficial effects of the present disclosure more clearly understood, the present disclosure is further described in detail below with reference to the accompanying drawings and embodiments. It should be understood that the specific embodiments described herein are merely used to interpret the present disclosure and are not intended to limit the present disclosure.

In an embodiments of the present disclosure, a bifunctional acidic catalyst includes choline chloride, Brønsted acid, and Lewis acid in a molar ratio of 1:1:(1-4), the bifunctional acidic catalyst being in a deep eutectic state.

The present disclosure also discloses a preparation method of furfural via catalyzing hemicellulose or xylose, including: adding raw material of hemicellulose or xylose and the bifunctional acidic catalyst as an aqueous phase to a reactor, followed by adding an oil phase extractant, generating furfural in the reactor through catalytic dehydration and hydrolysis reaction, and continuously extracting the furfural from the aqueous phase during the reaction process by the oil phase extractant until the reaction is ended.

The final aqueous phase generated after the reaction includes the bifunctional acidic catalyst, a small amount of unreacted raw materials including hemicellulose solution or xylose solution, and the final oil phase includes furfural, the oil phase extractant, etc.

After the reaction, the entire reaction system in the reactor exhibits a biphasic separation into an aqueous phase and an oil phase. The aqueous phase (including the bifunctional acidic catalyst) and the oil phase are separated via a separatory funnel. The separated aqueous phase is recycled directly, achieving zero loss of the bifunctional acidic catalyst. The recovered bifunctional acidic catalyst is then recombined with fresh oil phase to form a new biphasic reaction system, which undergoes the catalytic reaction following the procedures described above to prepare furfural. Therefore, both the aqueous phase and the catalyst in the preparation process may be recycled, which can reduce energy consumption and production cost.

The bifunctional acidic catalyst and the preparation method of furfural via catalyzing hemicellulose or xylose are further illustrated hereinafter by specific examples.

### Example 1

Preparation method of different ratios of bifunctional acidic catalysts included operations as follows.

S11, 1.39 g of choline chloride (ChCl), 0.9 g of oxalic acid (Ea), and 2.66 g of CrCl₃·6H₂O, with a molar ratio of 1:1:1, were weighed and placed into a screw-neck bottle, respectively, and heated at 70°C for 6 h to obtain a bifunctional acidic ionic deep eutectic solvent, which was denoted as a catalyst B.

S12, by changing the molar ratio in S11 to 1:1:0.5, 1:1:2, 1:1:3, and 1:1:4, bifunctional acidic ionic deep eutectic solvents were synthesized, which were denoted as catalysts A, C, D, and E.

S13, by replacing oxalic acid in S11 with malonic acid, succinic acid, and citric acid, respectively, with a molar ratio of 1:1:1, bifunctional acidic catalysts were synthesized, which were denoted as catalysts F, G, and H.

S14, by mixing choline chloride (ChCl) with oxalic acid (Ea) and CrCl₃·6H₂O in a molar ratio of 1:1, respectively, acidic eutectic catalysts were synthesized according to the S11, which were denoted as catalysts M and N.

### Example 2

The first preparation method of furfural via catalyzing hemicellulose or xylose included operations as follows.

S21, catalysts A, B, C, D, E, F, G, H, I, J, K, M, and N prepared in example 1 were added to a reactor of 25 mL, an addition amount of the catalysts was 20% of the mass of the aqueous phase, a volume ratio of the oil phase to the aqueous phase was 8:1, and a mass concentration of the added xylose was 0.15 g/mL (the mass concentration being on the basis of the mass of the water, the same hereinafter).

S22, the reactor was placed in an oil bath pot, heated to 140°C under magnetic stirring, and reacted for 90 min.

S23, after the reactor was cooled to room temperature, an upper oil phase and a lower aqueous phase were taken to detect the content of furfural and calculate the yield of furfural, respectively.

The yield of furfural (YFF) was determined when the amount of the added catalyst was 20% of the mass of the aqueous phase, as shown in Table 1 below.

**Table 1 Effect of catalysts with different ratios pf raw material on YFF (using xylose as substrate)**

| Number | A | B | C | D | E | F | G | H | I | J | K | M | N |
|---|---|---|---|---|---|---|---|---|---|---|---|---|---|
| YFF (%) | 64. 1 | 87. 0 | 72. 6 | 68. 7 | 70. 3 | 39. 0 | 42. 7 | 43. 3 | 51. 8 | 44. 0 | 46. 9 | 54. 7 | 51. 8 |

As can be seen from Table 1, the YFF is the highest at 87.0% when using the catalyst B.

### Example 3

The second preparation method of furfural via catalyzing hemicellulose or xylose included operations as follows.

S31, the catalyst B prepared in example 1 was taken and added to a reactor, an addition amount of the catalyst B was 20% of the mass of the aqueous phase, a volume ratio of methyl isobutyl ketone (MIBK) to water was 8:1, and the mass concentration of the added xylose was 0.15 g/mL.

S32, the reactor was placed in an oil bath pot, heated to 140°C-160°C under magnetic stirring, and reacted for 90 min.

S33, after the reactor was cooled to room temperature, an upper oil phase and a lower aqueous phase were taken to detect the content of furfural and calculate the YFF, respectively using the same manner in example 2.

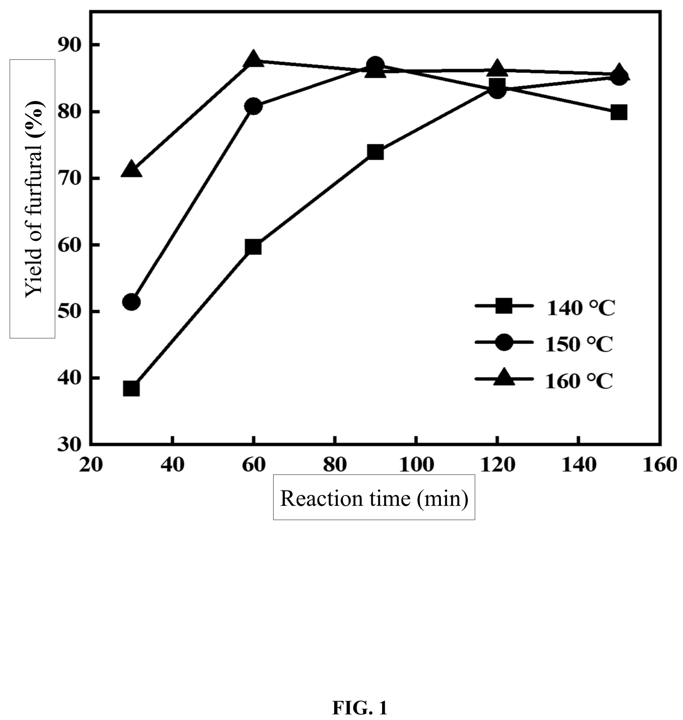

The detection results were shown in FIG. 1. It can be seen from FIG. 1 that when the reaction temperature is 140°C, 150°C, and 160°C, the YFF is 73.9%, 87.0%, and 86.0%, respectively; and when the reaction temperature is 150°C, the YFF is the highest at 87.0%.

### Example 4

The third preparation method of furfural via catalyzing hemicellulose or xylose included operations as follows.

S41, the catalyst B prepared in example 1 was taken and added to the reactor, an addition amount of the catalyst B was 20% of the mass of the aqueous phase, a volume ratio of MIBK to water was 8:1, and the mass concentration of the added xylose was 0.15 g/mL.

S42, the reactor was placed in an oil bath pot, heated to 150°C under magnetic stirring, and reacted for 30 min, 60 min, 90 min, 120 min, and 150 min, respectively; and after the reactor was cooled to room temperature, the upper oil phase and the lower aqueous phase were taken to detect the content of furfural and the YFF, respectively, using the same manner in example 2.

The detection results were shown in FIG. 1. It can be seen from FIG. 1 that the YFF were 51.4%, 80.8%, 87.0%, 83.2%, and 85.2% when the reaction time is 30 min, 60 min, 90 min, 120 min, and 150 min, respectively; and when the reaction time is 90 min, the YFF is the highest at 87.0%.

### Example 5

The fourth preparation method of furfural via catalyzing hemicellulose or xylose included operations as follows.

S51, the catalyst B prepared in example 1 was weighed according to a mass concentration of 0.05 g/mL, 0.10 g/mL, 0.15 g/mL, 0.20 g/mL, and 0.25 g/mL, respectively, and added to a reactor of 25 mL, a volume ratio of MIBK to water was 8:1, and a mass concentration of the added xylose was 0.15 g/mL.

S52, the reactor was placed in an oil bath pot, heated to 150°C under magnetic stirring, and reacted for 90 min.

S53, after the reactor was cooled to room temperature, an upper oil phase and a lower aqueous phase were taken to detect the content of furfural and calculate the YFF, respectively using the same manner in example 2.

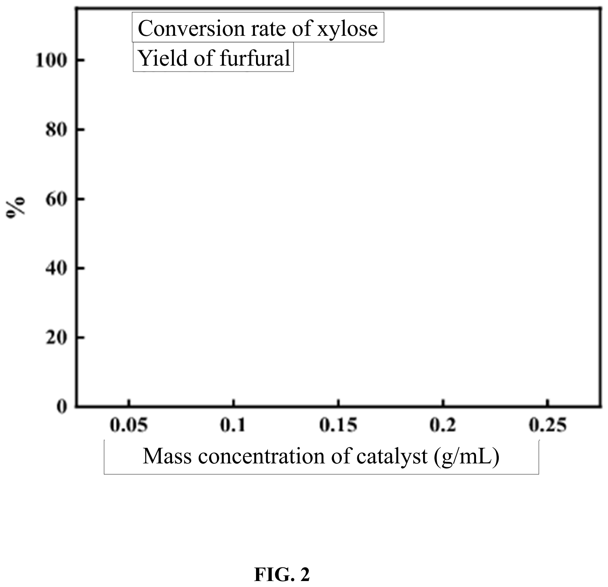

The detection results were as shown in FIG. 2. It can be seen form FIG. 2 that when the mass concentration of the catalyst B is 0.05 g/mL, 0.10 g/mL, 0.15 g/mL, 0.20 g/mL, and 0.25g/mL, respectively, the YFF is 75.8%, 82.9%, 83.0%, 87.0% and 87.1%, respectively. It is comprehensively considered that the optimal mass concentration of catalyst B is 0.20 g/mL, and the corresponding YFF is 87.0%.

### Example 6

The fifth preparation method of furfural via catalyzing hemicellulose or xylose included operations as follows.

S61, the catalyst B prepared in example 1 was taken and added to the reactor, a mass concentration of the catalyst B was 0.20 g/mL, and a volume ratio of MIBK to water was 8:1.

S62, xylose was weighed according to a mass concentration of 0.05 g/mL, 0.10 g/mL, 0.15 g/mL, 0.20 g/mL, and 0.25 g/mL, respectively, and added to the reactor; and the reactor was placed in an oil bath pot, heated to 150°C under magnetic stirring, and reacted for 90 min.

S63, after the reactor was cooled to room temperature, an upper oil phase and a lower aqueous phase were taken to detect the content of furfural and calculate the YFF, respectively using the same manner in example 2.

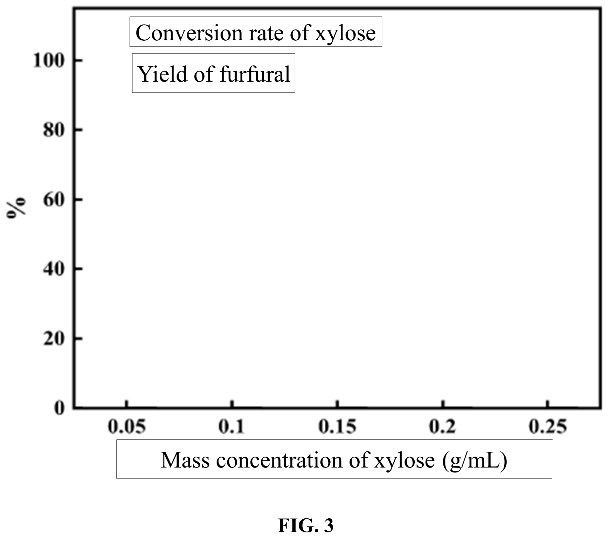

The detection results were shown in FIG. 3. It can be seen from FIG. 3 that when the mass concentration of xylose is 0.05 g/mL, 0.10 g/mL, 0.15 g/mL, 0.20 g/mL, and 0.25 g/mL, respectively, the YFF is 80.9%, 83.7%, 87%, 80%, and 79%, respectively; and when the mass concentration of xylose is 0.15 g/mL, the YFF is the highest at 87.0%.

### Example 7

The sixth preparation method of furfural via catalyzing hemicellulose or xylose included operations as follows.

S71, the catalyst B prepared in example 1 was taken and added to the reactor, the mass concentration of the catalyst B was 0.20 g/mL, and the mass concentration of the added xylose was 0.15 g/mL.

S72, MIBK and water were added to the reactor according to a volume ratio of 2:1, 4:1, 6:1, 8:1, and 10:1, respectively, and the reactor was placed in an oil bath pot, heated to 150°C under magnetic stirring, and reacted for 90 min.

S73, after the reactor was cooled to room temperature, an upper oil phase and a lower aqueous phase were taken to detect the content of furfural and calculate the YFF, respectively using the same manner in example 2.

It is determined that the YFF is 81.8%, 83.3%, 85.5%, 87%, and 85.9% when the volume ratio of MIBK to water are 2:1, 4:1, 6:1, 8:1, and 10:1, respectively. When the volume ratio of the oil phase to the aqueous phase is 8:1, the YFF is the highest at 87.0%.

### Example 8

The seventh preparation method of furfural via catalyzing hemicellulose or xylose included operations as follows.

S81: the catalyst B prepared in example 1 was taken and added to the reactor, a mass concentration of the catalyst B was 0.20 g/mL, a mass concentration of the added xylose was 0.15 g/mL, the volume ratio of the oil phase to the aqueous phase was 8:1, and oil phase was MIBK, toluene, tetrahydrofuran, acetophenone, valerolactone, n-butanol, tetrahydronaphthalene, and ethyl acetate, respectively.

S82, the reactor was placed in an oil bath pot, heated to 150°C under magnetic stirring, and reacted for 90 min.

S83, after the reactor was cooled to room temperature, an upper oil phase and a lower aqueous phase were taken to detect the content of furfural and calculate the YFF, respectively using the same manner in example 2.

It is determined that the YFF is 87.0%, 78.1%, 76%, 66%, 53.2%, 49.9%, 69.4%, and 60.2%, respectively, when the oil phase is MIBK, toluene, tetrahydrofuran, acetophenone, valerolactone, n-butanol, tetrahydronaphthalene, and ethyl acetate, respectively. When the oil phase was MIBK, the YFF is the highest at 87.0%.

### Example 9

The eighth preparation method of furfural via catalyzing hemicellulose or xylose included operations as follows.

S91, the catalyst B prepared in example 1 was taken and added to the reactor, the mass concentration of the catalyst B was 0.20 g/mL, a mass concentration of the added hemicellulose was 0.15 g/mL, and a volume ratio of the oil phase to the aqueous phase was 8:1.

S92, the reactor was placed in an oil bath pot, heated to 140°C-160°C under magnetic stirring, and reacted for 90 min.

S93, after the reactor was cooled to room temperature, an upper oil phase and a lower aqueous phase were taken to detect the content of furfural and calculate the YFF, respectively using the same manner in example 2.

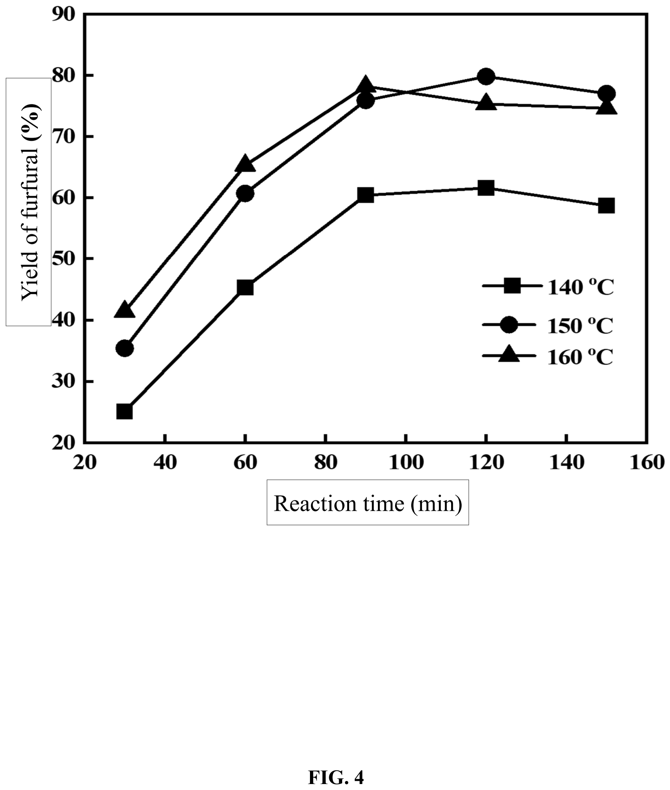

The detection results were as shown in FIG. 4. It can be seen from FIG. 4 that when the reaction temperature is 140°C, 150°C, and 160°C, the YFF is 60.4%, 79.8%, and 78.2%, respectively; and when the reaction temperature is 150°C, the YFF is the highest at 79.8%.

### Example 10

The ninth preparation method of furfural via catalyzing hemicellulose or xylose included operations as follows.

S101, the catalyst B prepared in example 1 was taken and added to the reactor, the mass concentration of the catalyst B was 0.20 g/mL, and a mass concentration of the added hemicellulose was 0.15 g/mL. A volume ratio of the oil phase to the aqueous phase was 8:1.

S102, the reactor was placed in an oil bath pot, heated to 150°C under magnetic stirring, and the reacted for 30 min-150 min.

S103, an upper oil phase and a lower aqueous phase were taken to detect the content of furfural and calculate the YFF, respectively using the same manner in example 2.

The detection results were determined as shown in FIG. 4. It can be seen from FIG. 4 that when the reaction time is 30 min, 60 min, 90 min, 120 min, and 150 min, respectively, the YFF is 35.4%, 60.7%, 79.8%, 78.2%, and 77%, respectively; and when the reaction time is 90 min, the YFF is the highest at 79.8%.

### Example 11

The tenth preparation method of furfural via catalyzing hemicellulose or xylose included operations as follows.

S111, hemicellulose was weighed according to a mass concentration of 0.10 kg/L, 0.15 kg/L, 0.2 kg/L, 0.25 kg/L, 0.3 kg/L, 0.35 kg/L, and 0.4 kg/L, respectively, and added to the reactor. The volume ratio of the oil phase to the aqueous phase was 8:1, and the mass concentration of the catalyst B prepared in example 1 was 0.20 g/mL.

S112, the reactor was placed in an oil bath pot, heated to 150°C under magnetic stirring, and reacted for 90 min.

S113, an upper oil phase and a lower aqueous phase were taken to detect the content of furfural and calculate the YFF, respectively using the same manner in example 2.

The yield of furfural prepared by dehydration of hemicellulose with different mass concentrations was determined and shown in Table 2.

**Table 2 Effect of hemicellulose with different mass concentrations on YFF (using hemicellulose as substrate)**

| | | | | | | | |
|---|---|---|---|---|---|---|---|
| Mass concentration (g/mL) | 0.10 | 0.15 | 0.2 | 0.25 | 0.3 | 0.35 | 0.4 |
| YFF (%) | 75.1 | 79.8 | 74.9 | 73.4 | 68.7 | 63.8 | 63.4 |

As can be seen from Table 2, when furfural is prepared by dehydration of hemicellulose at the mass concentration of 0.15 g/mL, the YFF is the highest at 79.8%.

### Example 12

The eleventh preparation method of furfural via catalyzing hemicellulose or xylose included operations as follows.

S121, the catalyst B prepared in example 1 was weighed according to a mass concentration of 0.05 g/mL, 0.10 g/mL, 0.15 g/mL, 0.20 g/mL, and 0.25 g/mL, respectively and added to a reactor of 25 mL. A volume ratio of MIBK to water was 8:1, and a mass concentration of the added hemicellulose was 0.15 g/mL.

S122, the reactor was placed into an oil bath pot, heated to 150°C under magnetic stirring, and reacted for 90 min.

S123, an upper oil phase and a lower aqueous phase were taken to detect the content of furfural and calculate the YFF, respectively using the same manner in example 2.

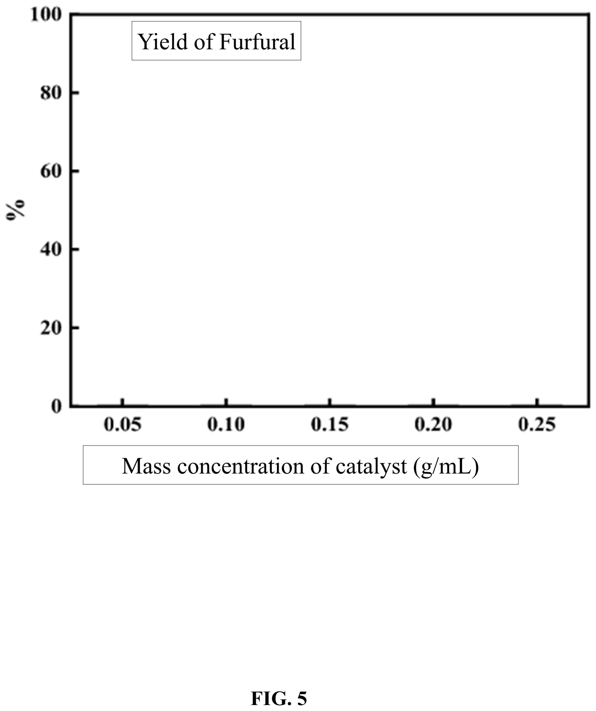

The detection results were shown in FIG. 5. It can be seen from FIG. 5 that when the mass concentration of catalyst B is 0.05 g/mL, 0.10 g/mL, 0.15 g/mL, 0.20 g/mL, and 0.25 g/mL, respectively, the YFF was 50.6%, 64.4%, 75.6%, 79.8%, and 76.8%, respectively. It is comprehensively considered that the optimal mass concentration of catalyst B is 0.20 g/mL, and the corresponding YFF is 87.0%.

### Example 13

The twelfth preparation method of furfural via catalyzing hemicellulose or xylose included operations as follows.

S131, the catalyst B prepared in example 1 was taken and added to the reactor, a mass concentration of the catalyst B was 0.20 g/mL, a mass concentration of xylose was 0.15 g/mL, and a volume ratio of MIBK to water was 8: 1.

S132, the reactor was placed in an oil bath pot, heated to 150°C under magnetic stirring, and reacted for 90 min.

S133, after the rector was cooled to room temperature, an upper oil phase and a lower aqueous phase were taken to detect the content of furfural and calculate the YFF, respectively using the same manner in example 2.

After the reaction is ended, the whole reaction system in the reactor exhibited two-phase separation of aqueous phase and oil phase. The aqueous phase (containing catalyst) and oil phase were separated by a separatory funnel, and the separated aqueous phase was recycled to form a new two-phase reaction system with the fresh oil phase. The catalytic reaction was carried out repeatedly according to the above operations 131-133 to prepare furfural, and the YFF was determined for each cycle.

It is determined that when the number of cycles of the deep eutectic catalyst is 0, 1, 2, 3, 4, 5, 6, 7, and 8, respectively, the YFF is 87.0%, 88.7%, 88.5%, 87.6%, 85.2%, 86.8%, 85.3%, 86.5%, and 85.7%. It may be found that there is almost no significant loss of activity after recycling the deep eutectic catalyst for 8 cycles.

The above description merely represents the preferred embodiments of the present disclosure and shall not be construed as limiting the present disclosure. Any modifications, equivalent substitutions, improvements, etc., made within the spirit and principle of the present disclosure shall be included within the scope of protection of the present disclosure.

## Claims

1. A bifunctional acidic catalyst, comprising choline chloride, Brønsted acid, and Lewis acid in a molar ratio of 1:1:(1-4), wherein the bifunctional acidic catalyst is in a deep eutectic state.

2. The bifunctional acidic catalyst of claim 1, wherein the Brønsted acid is any one of oxalic acid, malonic acid, succinic acid, and citric acid.

3. The bifunctional acidic catalyst of claim 1, wherein the Lewis acid is any one of CrCl₃·6H₂O, AlCl₃·6H₂O, SnCl₄·5H₂O, and FeCl₃·6H₂O.

4. The bifunctional acidic catalyst of claim 1, wherein the bifunctional acidic catalyst is a deep eutectic solvent in the deep eutectic state, wherein the deep eutectic solvent is prepared by heating a mixture of 1 mol choline chloride, 1 mol oxalic acid, and 1 mol CrCl₃·6H₂O at 70°C for 6 h.

5. A preparation method of furfural via catalyzing hemicellulose or xylose, comprising:
adding the hemicellulose or the xylose and the bifunctional acidic catalyst of any one of claims 1-4 as an aqueous phase into a reactor, followed by adding an oil phase extractant into the reactor;
generating the furfural in the reactor through catalytic dehydration and hydrolysis reaction; and
continuously extracting the furfural from the aqueous phase by the oil phase extractant during a reaction process until the reaction is ended.

6. The preparation method of claim 5, wherein the oil phase extractant includes any one of methyl isobutyl ketone, toluene, tetrahydrofuran, valerolactone, n-butanol, acetophenone, ethyl acetate, and tetrahydronaphthalene.

7. The preparation method of claim 5, wherein a mass concentration of the hemicellulose or xylose in the aqueous phase is within a range of 0.05 g/mL-0.25 g/mL.

8. The preparation method of claim 5, wherein a mass concentration of the bifunctional acidic catalyst in the aqueous phase is within a range of 0.05 g/mL-0.25 g/mL.

9. The preparation method of claim 5, wherein an amount of the aqueous phase is 1/2 to 1/10 of an amount of the oil phase extractant.

10. The preparation method of claim 5, wherein a reaction temperature of the catalytic dehydration and the hydrolysis reaction is within a range of 140°C-160°C, and a reaction time of the catalytic dehydration and the hydrolysis reaction is within a range of 30 min-150 min.
